# EUROPÄISCHE PATENTSCHRIFT

(11) **EP 4 655 065 B1**
(45) Veröffentlichungstag und Bekanntmachung des Hinweises auf die Patenterteilung: **12.08.2026**
(21) Anmeldenummer: 24708974.1
(22) Anmeldetag: 21.02.2024
(51) Int. Cl.: A61N 2/02, A61B 34/20, A61N 7/00, A61B 90/00, A61N 2/00, A61B 18/14, A61B 34/10

(54) **VORRICHTUNG ZUR NICHT-INVASIVEN NEUROSTIMULATION**
APPARATUS FOR NON-INVASIVE NEUROSTIMULATION
DISPOSITIF DE NEUROSTIMULATION NON INVASIVE

(30) Priorität: 27.02.2023 DE 102023104761
(43) Veröffentlichungstag der Anmeldung: 03.12.2025
(73) Patentinhaber: FORBENCAP GmbH, 87527 Sonthofen (DE)
(72) Erfinder: GÖTZ, Stefan, 85659 Forstern (DE)
(74) Vertreter: advotec.
(86) Internationale Anmeldenummer: PCT/EP2024/054385
(87) Internationale Veröffentlichungsnummer: WO 2024/179897

(56) Entgegenhaltungen:
- EP-A1- 1 270 043
- WO-A1-2023/278199
- US-A- 5 565 892
- US-A1- 2013 345 718
- US-A1- 2018 303 559
- US-A1- 2019 142 401
- US-A1- 2021 008 382
- US-A1- 2022 346 894

## Beschreibung

Die Erfindung betrifft eine Vorrichtung zur nicht-invasiven Neurostimulation.

### Stand der Technik

Neuronen, auch bekannt als Nervenzellen, werden stimuliert durch elektrische Stimuli, die sich anschließend entlang der Zellmembranen fortbewegen. Diese Impulse werden alternativ zu elektrischen Stimuli auch beispielsweise von Neurotransmittern, chemischen Botenstoffen, ausgelöst, die von einer Zelle auf die nächste über Synapsen übertragen werden. Dies führt zur Aktivierung von Rezeptoren in der Zellmembran und zur Weiterleitung des Impulses entlang des Neurons.

Ein Schaltkreis im Gehirn bezieht sich dabei vorzugsweise auf eine Gruppe von Neuronen, die zusammenarbeiten, um eine bestimmte Aufgabe und/oder Funktion auszuführen. Diese Neuronen sind miteinander verbunden und bilden ein Netzwerk, das Signale durch die Synapsen weiterleitet. Es gibt verschiedene Arten von Schaltkreisen im Gehirn, die für unterschiedliche Funktionen verantwortlich sind, wie z. B. die Wahrnehmung, Bewegung, Gedächtnis, Emotionen und kognitiver Prozesse. Die Schaltkreise im Gehirn können sich im Laufe der Zeit verändern und anpassen, was als neuroplastische Veränderung bezeichnet wird. Dies ermöglicht das Lernen und die Anpassung an neue Umgebungen und Erfahrungen.

Es gibt in der Medizin verschiedene Arten zur insbesondere nicht-invasiven Aktivierung und/oder Stimulation einzelner Neuronen oder ganzer Schaltkreise.

Die (transkranielle) Magnetstimulation (TMS) ist eine Technik zur nicht-invasiven Aktivierung bzw. Stimulation von Neuronen, z. B. im Gehirn des bewussten bzw. sinneswachen Menschen, die insbesondere verwendet wird, um gezielte Bereiche des Gehirns zu stimulieren, indem sie kurze, präzise Magnetfelder erzeugt. Diese Felder erzeugen elektrische Ströme in den Neuronen, die die Aktivität dieser Zellen beeinflussen und dadurch bestimmte Funktionen des Gehirns beeinflussen können. Diese Technik kann also Signale in Neuronen "schreiben" und diese dazu bringen, ihrerseits aktiviert zu werden, so dass das Gehirn diese Signale wie physiologische bzw. natürliche vom Körper generierte Signale verarbeitet.

TMS verwendet in der Regel kurze, starke Strompulse in einer oder mehreren Stimulationseinrichtungen, typischerweise einer oder mehreren magnetischen Spulen. Die Strompulse umfassen typischerweise eine Stromstärke über 1000 A oder sogar über 5000 A. Die Strompulse haben oftmals eine Gesamtpulsdauer zwischen 5 µs und 100 ms, bevorzugt zwischen 50 µs und 1 ms. Die Strompulse sind geeignet, um induzierte elektrische Feldstärken meist zwischen 100 mV/m und 2000 V/m, bevorzugt zwischen 10 V/m und 200 V/m in einem Körpergewebe zu erzeugen. Induzierte elektrische Felder können wiederum mit Körperzellen, insbesondere Neuronen wechselwirken und insbesondere deren elektrisches Potential verschieben (sogenannte Polarisation), was die Erregbarkeit für endogene Signale eines neuronalen Schaltkreises beeinflussen kann, und/oder bei hohen Stärken elektrische Signale als Reaktion, sogenannte Aktionspotentiale auslösen. Meist werden Stimulationseinrichtungen mit einer bestimmten räumlichen elektrischen Feldverteilung im Körper, bevorzugt sogenannte fokale Spulen verwendet, die eine genaue Positionierung relativ zum Körper benötigen, um Felder in bestimmte Strukturen, beispielsweise anatomisch oder funktional definierte Strukturen zu erzeugen. Fokussierte Ultraschallstimulation setzt als Alternative als zumindest eine Stimulationseinrichtung meist Ultraschall-Erzeuger, -koppler oder - Transducer ein, die vorzugsweise gerichtete Schallwellen abgeben. Bei der Verwendung mehrerer Stimulationseinrichtungen lässt sich eine akustische oder elektromagnetische Wellenfeldsynthese nach dem Stand der Technik erreichen.

TMS wird vor allem in der neurologischen und/oder psychiatrischen Forschung eingesetzt, um die Wirkung von Stimulation auf die Gehirnfunktion zu untersuchen. Es wurde jedoch auch als therapeutische Behandlungsmethode für bestimmte Erkrankungen wie Depressionen, Schizophrenie, Migräne und Schmerzen untersucht.

Darüber hinaus können bestimmte Rhythmen und Muster, wie die so genannte repetitive TMS (rTMS) und/oder die Theta-Burst-Stimulation und/oder die Quadripuls-Stimulation, verändern, wie ein Schaltkreis endogene Signale anderer Neuronen verarbeitet, z. B. durch Verschiebung der so genannten Erregbarkeit eines Schaltkreises. Sogenannte hemmende Paradigmen können einen Schaltkreis weniger erregbar machen, wohingegen erregende Paradigmen die Sensitivität eines betreffenden Schaltkreises erhöhen. Der zugrunde liegende Effekt wird Neuromodulation genannt.

Die Neuromodulation ermöglicht es beispielsweise, das Gleichgewicht zwischen gegenläufigen Schaltkreisen im Gehirn zu verschieben oder bestimmte Schaltkreise für eine begrenzte Dauer praktisch "auszuschalten". Die TMS ist daher zu einem wichtigen Instrument in den experimentellen und/oder anwendungsbezogenen Neurowissenschaften geworden. Darüber hinaus bildet die Neuromodulation die Grundlage für eine Reihe von medizinischen Behandlungsverfahren.

TMS aktiviert Neuronen und moduliert die neuronale Kommunikation im Gehirn durch elektromagnetische Induktion mit starken kurzen Magnetfeldern. Sie ist für die Behandlung einer Reihe von Erkrankungen zugelassen, wird für die neurologische Diagnose eingesetzt und ist in Kombination mit der Bildgebung zu einem Schlüsselinstrument in der experimentellen Hirnforschung geworden. Die therapeutische Wirkung der TMS ist jedoch mäßig und variabel, mit Remissionsraten bei Depressionen von 14-33 %, und die neuromodulatorische Wirkung, die auch in der experimentellen Hirnforschung eingesetzt wird, ist relativ schwach und oftmals variabel. Als ein zentraler Grund für die mäßige Effektstärke ist dabei vor allem das Handling durch die Operatorin oder den Operator.

Der wissenschaftliche Zeitschriftenartikel Goetz und Deng von 2017 [Goetz, S. M., & Deng, Z. D. (2017). The development and modelling of devices and paradigms for transcranial magnetic stimulation. International Review of Psychiatry, 29(2), 115-145. doi: 10.1080/09540261.2017.1305949] beschreibt hierbei insbesondere den Stand der Technik hinsichtlich der verfügbaren Spulen- und Pulsquellentechnologie, sowie die erreichbare Fokalität.

Aus der insbesondere experimentellen Anwendung ist es bekannt, eine zur TMS verendete Magnetspule beispielsweise mithilfe eines Roboters oder manuell relativ zu einem Kopf eines Probanden zu positionieren. Die Spule wird dabei meist durch optische Stereotaxie mit zwei Stereokameras verfolgt, wobei die Stereokameras vorzugsweise an der Spule angebrachte optische, insbesondere retroreflektierende Marker, beispielsweise Kugeln, im Raum lokalisieren, um daraus eine Position eines Spulenfokus relativ zu einer Hirnanatomie anzuzeigen. Diese Stereotaxie, wie sie auch aus der Neurochirurgie und der minimalinvasiven Chirurgie bekannt ist, ist optional, und kann grundsätzlich auch durch eine manuelle Positionierung durch einen Anwender ersetzt werden. Ebenso können Positionen und/oder Orientierungen im Raum auch über magnetische und/oder allgemein elektromagnetische Messung, beispielsweise Triangulation gemessen werden.

TMS aktiviert Neuronen sehr fokussiert, so dass die Stimulationsspule mit sechs Freiheitsgraden (englisch degree of freedom, kurz DOF), insbesondere drei Positionskoordinaten und drei Orientierungskoordinaten möglichst genau positioniert werden muss. Drei der Freiheitsgrade sind durch die Kopfoberfläche und damit den Knochenaufbau des Schädels eingeschränkt, da die TMS-Spule den Kopf Patienten im Fokuspunkt berühren sollte. Folglich kann die Spule um die Oberflächennormale des Berührungspunkts gedreht (1 DOF) und um den Fokuspunkt seitlich und/oder nach vorne und/oder nach hinten verschoben werden (2 DOF), um verschiedene Schaltkreise zu stimulieren. Der Bediener muss jedoch darauf achten, dass die Spule dabei den Kopf stets perfekt berührt, sodass sich der Fokuspunkt der Spule über einem gewünschten kortikalen Ziel befindet und dabei die Ausrichtung der Spule zur Zielanvisierung innerhalb des Gehirns perfekt ist. Viele Bediener, insbesondere unerfahrene Bediener, haben damit erhebliche koordinative Probleme, da mehrere Freiheitsgrade bei der Positionierung der Spule parallel zueinander abgestimmt und/oder durch entsprechende multidirektionale Gegenbewegungen der Spule kompensiert werden müssen. Obwohl Anwender eine Links-Rechts-Ausrichtung um einen Fokuspunkt vergleichsweise gut kontrollieren können, neigen ebendieselben Anwender dazu, einen Gierausgleich und/oder Verkippungsausgleich, betrachtet um eine Spulenlängsachse, nur schwer zu kontrollieren. Darüber hinaus muss sich der Anwender der TMS-Spule fortlaufend und nicht nur temporär mit einer korrekten Positionierung der Spule relativ zu dem Kopf des Patienten befassen, um derart einen bestimmten neuronalen Schaltkreis anzusteuern. Dies erschwert die Problematik zusätzlich, da der Anwender zur korrekten Positionierung stets vollumfängliche Konzentration aufbringen muss, was insbesondere bei längerwierigen Behandlungen problematisch sein kann.

Eine Technologie, die ein Positionieren der Spule relativ zu dem anzuvisierenden Fokuspunkt erleichtert, wäre zur Überwindung der Problematik äußerst hilfreich.

Zu zumindest teilweisen Überwindung des Positionierungsproblems sind bereits Ansätze bekannt. Beispielsweise wird ein Anwender heutzutage in der Regel durch insbesondere rahmenlose Stereotaxiesysteme unterstützt, meist optische und/oder elektromagnetische Systeme, die die Position und Ausrichtung der Spule in den sechs zu kontrollierenden Freiheitsgraden (6 DOF) sowie die entsprechenden Freiheitsgrade des Kopfes des Patienten (6 DOF) überwachten, um die Position der Spule relativ zu dem Kopf vorzugsweise in Echtzeit anzuzeigen. Wenn zusätzlich individuelle Gehirnscans oder Standard-Gehirnmodelle (z. B. MNI-Modell, benannt nach dem Montreal Neurological Institute) des Patienten verfügbar sind, kann eine Neuronavigationssoftware das Gehirn des Patienten und die erwartete Position der Spule über der Gehirnfaltung zusätzlich anzeigen. Die wissenschaftliche Referenz Goetz und Kammer [S. Goetz, T. Kammer (2023). Neuronavigation. In: Oxford Handbook of Transcranial Stimulation. Second edition, Oxford University Press] diskutiert den Stand der Technik der Neuronavigation für die nichtinvasive Hirnstimulation.

Diese Neuronavigationssysteme werden vom Anwender jedoch oftmals als unhandlich empfunden, da der Anwender einen rechenstarken Computer und zusätzlich einen möglichst großen Bildschirm benötigt, um die von dem Gehirn und der Positionierungseinrichtung erzeugten, insbesondere miteinander überlagerten Bilder auch aus der Ferne, d. h. von dem Behandlungsort aus, zu erkennen. Der Anwender sieht dabei ein anzuvisierendes Ziel innerhalb eines Gehirnscans mittels funktioneller Bildgebung beispielsweise als gefärbte Wolke und zusätzlich hierzu einen virtualisierten Fokussierpunkt der Spule relativ zu der Oberfläche des Kopfes als ebenfalls farbiger Marker, der sich bewegt, wenn der Anwender die Ausrichtung der Spule koordiniert.

Hierbei ist, ähnlich wie bei virtualisierten Operationsvorgängen und Teleoperationen, ein hohes Maß an Erfahrung und Übung des Anwenders erforderlich, um zum einen die Positionierung der Spule mit ihren sechs Freiheitsgraden manuell oder ggf. roboterunterstützt relativ zu dem Kopf des Patienten zu koordinieren, während sämtliche notwendigen Positionsinformationen nicht von der Spule, sondern dem Bildschirm geliefert werden. Auf diesem Bildschirm werden dabei auch Positionskorrekturinformationen in Rückkopplung angezeigt, die von dem Anwender dann umgesetzt werden müssen. Für den Anwender ist eine derartige Positionierung ungefähr so schwierig, wie ein Versuch, einen bestimmten Punkt auf dem eigenen Hinterkopf unter Betrachtung desselben Punktes durch zwei Spiegel zu berühren.

Mithin überfordert das bekannte Positionierungsverfahren nicht nur unerfahrene Anwender, sondern erfordert selbst von diesen eine besonders hohes Maß an Aufmerksamkeit, so dass eine schnelle Erschöpfung des Anwenders eintritt. In der Konsequenz sind keine längeren Behandlungen möglich.

Ähnliche Positionierungsproblematiken bestehen vor Vorrichtungen zur Stimulation von Neuronen und/oder neuronalen Schaltkreisen mittels fokussiertem Ultraschall (englisch focussed ultrasound stimulation, kurz FUS). Dabei handelt es sich um eine Neurostimulationstechnik, die sich momentan noch im Forschungsstadium befindet, sich jedoch dazu eignet, tiefere, subkortikale Ziele mit einer gewissen Fokalität zu aktivieren. Zur Beschleunigung des Forschungsfortgangs ist es daher wünschenswert, auch bei dieser neuartigen Technologie die bestehenden Positionierungsprobleme zumindest teilweise zu überwinden.

Aus der US2021/008382A1 ist eine Vorrichtung zur nicht-invasiven Neurostimulation bekannt.

Der Erfindung liegt somit die Aufgabe eine Vorrichtung für die nicht-invasive Stimulation von Neuronen derart weiterzuentwickeln, dass insbesondere die aus dem Stand der Technik bekannten Positionierungsschwierigkeiten derartiger Vorrichtungen relativ zu einem Kopf eines Patienten zumindest teilweise gelöst werden.

Die Aufgabe wird gelöst durch eine Vorrichtung zur nicht-invasiven Neurostimulation gemäß den Merkmalen des Patentanspruchs 1.

### Offenbarung der Erfindung

Erfindungsgemäß ist eine insbesondere mobile Vorrichtung zur nicht-invasiven Neurostimulation vorgeschlagen. Die bevorzugt mobile Vorrichtung zur nicht-invasiven Neurostimulation umfasst eine Stimulationseinrichtung, die besonders bevorzugt ausgebildet ist, relativ zu einem Kopf eines Patienten positionierbar zu sein, und insbesondere mindestens ein Stimulationssignal zu erzeugen, durch das mindestens ein Neuron, vorzugsweise eine Vielzahl von Neuronen, und/oder einen neuronalen Schaltkreis anregbar ist. Die Stimulationseinrichtung wird auch als Stimulationsaktuator bezeichnet.

Nicht von der Erfindung umfasst, ist eine chirurgische Vorrichtung vorgeschlagen. Die chirurgische Vorrichtung umfasst vorzugsweise ein chirurgisches Instrument. Ein derartiges chirurgisches Instrument kann beispielsweise einen Skalpell und/oder eine Pinzette und/oder eine Schere und/oder eine Klemme und/oder ein Nadel und/oder einen Trokar und/oder einen Bohrer und/oder eine Säge und/oder einen Retraktor und/oder einen Dilatator und/oder ein elektrochirurgisches Instrument umfassen.

Die Vorrichtung zur nicht-invasiven Neurostimulation umfasst eine Anzeigevorrichtung, die dazu eingerichtet ist, mindestens eine Bildinformation, so anzuzeigen, dass ein Anwender und/oder ein Bediener vorzugsweise eine zusätzliche Information vorzugsweise in Form der Bildinformation passend für ihre/seine Augenposition und/oder Blickrichtung erhält. Durch die mindestens eine Bildinformation wird vorzugsweise das durch die Stimulationseinrichtung und/oder die in der Sichtachse des Anwenders liegende anatomische Struktur und/oder das Gewebe und/oder der Körperbereich und/oder andere Gegenstände und/oder sonstige funktionelle Informationen unter der Stimulationseinrichtung virtuell sichtbar gemacht. Dadurch wird der Anwender bei der Positionierung der Stimulationseinrichtung unterstützt.

Die mindestens eine Bildinformation umfasst vorzugsweise eine Gehirnscan-Information und/oder eine generische Gehirndateninformation über ein Gehirn des Patienten. Die mindestens eine Bildinformation umfasst vorzugsweise eine Anatomie-Information und/oder eine generische anatomische und/oder funktionale und/oder physiologische Körperinformation über einen Körperteil eines Patienten. Grundsätzlich ist es beliebig, um welchen Körperteil es sich handelt. Bevorzugt handelt es sich bei dem Körperteil um einen Kopf, insbesondere um ein Gehirn des Patienten.

Die bevorzugt mobile Vorrichtung zur nicht invasiven Neurostimulation kann insbesondere zur nicht-invasiven Nervenstimulation verwendet werden. In diesem Fall umfasst die Vorrichtung die Stimulationseinrichtung, die vorzugsweise ausgebildet ist, relativ zu einem Körperteil eines Patienten positionierbar zu sein, und besonders bevorzugt mindestens ein Stimulationssignal zu erzeugen, durch das mindestens eine Nervenzelle anregbar ist. Die Stimulationseinrichtung umfasst die Anzeigevorrichtung, die dazu eingerichtet ist, mindestens eine Bildinformation, insbesondere eine Nervensystem-Information und/oder eine generische Nervendateninformation, über ein Körperteil und/oder ein Nervensystem des Patienten anzuzeigen, um derart den Anwender bei der Positionierung der Stimulationseinrichtung zu unterstützen.

Der Erfinder hat ferner erkannt, dass der erfindungsgemäße Ansatz auch auf eine allgemeine mobile chirurgische Vorrichtung erstreckt werden kann, die eine Anzeigevorrichtung der erfindungsgemäße Art umfasst, die dazu eingerichtet ist, mindestens eine Bildinformation, insbesondere eine Gewebe- und/oder eine Knocheninformation und/oder eine Gehirnscan-Information und/oder eine Röntgeninformation über einen Körperteil des Patienten so anzuzeigen, dass ein Anwender und/oder ein Bediener vorzugsweise eine zusätzliche Information vorzugsweise in Form der Bildinformation passend für ihre/seine Augenposition und/oder Blickrichtung erhält, die beispielsweise das eigentlich verdeckte Gewebe und/oder andere Gegenstände und/oder sonstige funktionelle Informationen, die sich unterhalb der Vorrichtung im Körperinneren des Patienten befinden, zumindest virtuell sichtbar macht, um derart den Anwender bei der Positionierung der Vorrichtung zu unterstützen. Eine derartige chirurgische Vorrichtung (vorliegend nicht vom Schutzumfang umfasst) kann beispielsweise zur minimal-invasiven Chirurgie verwendet werden. Eine derartige chirurgische Vorrichtung kann alternativ oder ergänzend auch zu Trainingszwecken und/oder Übungszwecken verwendet werden, um eine zu trainierende Person durch die angezeigte Bildinformation zu unterstützen, bevor sie/er eine entsprechende Anwendung ohne Zusatzhilfe ausführt. Die mobile chirurgische Vorrichtung erhält die Bildinformation vorzugsweise durch einen C-Bogen und/oder durch eine anderes bildgebendes Medizingerät, die hierfür beispielsweise zuvor aufgenommen wurde und über geometrische Referenzpunkte am Körper, beispielsweise Marker, geometrisch mit der Szenerie referenziert oder registriert werden oder quasi zeitgleich aufgenommen wird. Die mobile chirurgische Vorrichtung erhält die Bildinformationen vorzugsweise online bzw. in Echtzeit oder offline aus zuvor gemachten Bild- und/oder Videoaufnahmen. Die erfindungsgemäße Stimulationseinrichtung ist eine besonders bevorzugte mobile chirurgische Vorrichtung zur nicht-invasiven Anwendung. Die mobile chirurgische Vorrichtung kann auch invasiv oder minimal-invasiv zur Anwendung kommen. Sämtliche Ausführungen erstrecken sich in gleicher Weise auf die mobile chirurgische Vorrichtung, da durch dieses die gleiche oder zumindest ähnliche technische Aufgabe gelöst wird. Die oben gennannte Stimulationseinrichtung kann entsprechend auch ein chirurgisches Instrument bezeichnen, insbesondere auch ein laparoskopisches Instrument. Ferner kann auch ein Inspektionsinstrument ein chirurgisches Instrument und somit eine Stimulationseinrichtung bezeichnen.

Die Anzeigevorrichtung umfasst vorzugsweise mindestens einen Bildschirm bzw. ein Display. Die Stimulationseinrichtung kann ein Gehäuse oder einen Gehäuseabschnitt umfassen, in dem mindestens ein Stimulator zur Erzeugung des mindestens einen Stimulationssignal angeordnet ist. Das Stimulationssignal ist vorzugsweise gepulst und/oder hochenergetisch. Die Stimulationseinrichtung ist vorzugsweise dazu ausgebildet, von dem Anwender manuell oder teilmanuelle, insbesondere roboterunterstützt relativ zu dem Körperteil, insbesondere dem Kopf, des Patienten positioniert zu werden. Die Stimulationseinrichtung oder das chirurgische Instrument kann beispielsweise einen Griffabschnitt umfassen, der von einem Anwender zur Positionierung der Stimulationseinrichtung oder des chirurgischen Instruments zumindest teilweise umgriffen werden kann. Die Stimulationseinrichtung oder das chirurgische Instrument kann auch an einem Roboterführungsarm angeordnet sein und mit Hilfe eines Griffabschnittes von dem Anwender an dem Roboterführungsarm relativ zu dem Körperteil platziert werden. Der Anwender koordiniert dabei zumeist sechs Freiheitsgrade der Stimulationseinrichtung oder des chirurgischen Instruments relativ zu dem Körperteil, insbesondere dem Kopf, des Patienten. Diese Koordination ist erfindungsgemäß durch die Anzeigevorrichtung vereinfacht.

Die Anzeigevorrichtung ist erfindungsgemäß an der Stimulationseinrichtung oder dem chirurgischen Instrument vorgesehen oder in dieser integriert. Dies ist ein wesentlicher Unterschied zum Stand der Technik, da dort eine Positionskorrekturhilfe beispielsweise auf einem Bildschirm angezeigt wird, der sich abseits, insbesondere beabstandet von der eigentlichen Stimulationseinrichtung oder dem chirurgischen Instrument befindet. Durch diese Beabstandung besteht im Stand der Technik allerdings ein großes Koordinierungsproblem für den Anwender, da er bei der Positionierung der Stimulationseinrichtung oder des chirurgischen Instruments Positionsinformationen verschiedener Quellen mit jeweils verschiedenen Bezugssystemen mental vereinheitlichen und untereinander koordinieren muss. Dies ist erfindungsgemäß nicht mehr der Fall, da die Anzeigevorrichtung vorzugsweise positionierungsunterstützende Informationen dem Anwender in dem Bezugssystem der Stimulationseinrichtung oder des chirurgischen Instruments anzeigt. Hierdurch ist die Koordinierungsproblematik, wie sie aus dem Stand der Technik bekannt war, erfindungsgemäß überwunden.

Während bekannte Stimulationseinrichtungen oder chirurgische Instrumente derzeit ohne jegliche Information darüber, was sich unter dem betreffenden Körperteil, insbesondere der Kopfhaut und dem Schädel befindet, relativ zu dem Körperteil des Patienten platziert werden, ermöglicht die erfindungsgemäße Ausführung einen zumindest virtuellen Durchblick bzw. eine virtuelle Durchsicht durch die Stimulationseinrichtung oder das chirurgische Instrument. Im Stand der Technik ist oftmals nicht einmal die Kopfhaut des Patienten ersichtlich, da diese durch Haare zumeist vollständig verdeckt ist. Erfindungsgemäß kann ein durch die Anzeigevorrichtung zumindest virtualisierter Blick auf das Gehirn und/oder andere gehirnbezogene Strukturen oder einen anderen Körperteil ermöglicht werden. So kann nur die Stimulationseinrichtung oder das chirurgische Instrument von dem Anwender während der Positionierung betrachtet werden, ohne dass eine Konzentration auf externe Quellen erforderlich ist. Hierdurch wird einer schnellen Ermüdung des Anwenders entgegengewirkt. Zudem kann der Anwender vorzugsweise Positionierungsinformationen über die Stimulationseinrichtung oder das chirurgische Instrument als geometrische Positionsinformationen zumindest visuell wahrzunehmen. Hierdurch wird dem Anwender vorzugsweise in Echtzeit ein Feedback über seine Genauigkeit bei der Positionierung der Stimulationseinrichtung oder des chirurgischen Instruments gegeben.

In einer bevorzugten Ausführungsform umfasst die Stimulationseinrichtung eine Magnetspulenvorrichtung mit mindestens einer Magnetspule zur insbesondere transkraniellen Magnetstimulation. Die Magnetspulenvorrichtung mit der mindestens einen Magnetspule ist ausgebildet, das mindestens eine Stimulationssignal als elektromagnetische Felder und/oder Wellen, durch die vorzugsweise ein fokussiertes elektromagnetisches Feld in das Gehirn des Patienten induziert wird, zu erzeugen. Die Magnetspulenvorrichtung umfasst rein optional zumindest einen Gehäuseabschnitt mit einer in Richtung des Kopfes orientierten Unterseite und einer in Richtung des Anwenders orientierten Oberseite, wobei die Anzeigevorrichtung vorzugsweise an der Oberseite vorgesehen oder in dieser integriert ist. Grundsätzlich ist es auch vorstellbar, dass die Anzeigevorrichtung an einer Seitenfläche des Gehäuseabschnittes angeordnet ist, was jedoch nicht so positive Auswirkungen auf die Positionierungsunterstützung hat, wie die Anordnung an der Oberseite des Gehäuseabschnittes. Die (transkranielle) Magnetstimulation (TMS) ist eine Technik zur nicht-invasiven Aktivierung bzw. Stimulation von Neuronen, z. B. im Gehirn des bewussten bzw. sinneswachen Menschen, die insbesondere verwendet wird, um gezielte Bereiche des Gehirns zu stimulieren, indem sie kurze, präzise Magnetfelder erzeugt. Diese Felder erzeugen elektrische Ströme in den Neuronen, die die Aktivität dieser Zellen beeinflussen und dadurch bestimmte Funktionen des Gehirns beeinflussen können. Diese Technik kann also Signale in Neuronen "schreiben" und diese dazu bringen, ihrerseits aktiviert zu werden, so dass das Gehirn diese Signale wie physiologische bzw. natürliche vom Körper generierte Signale verarbeitet.

In einer bevorzugten Ausführungsform ist die Stimulationseinrichtung dazu ausgebildet, das mindestens eine Stimulationssignal als insbesondere fokussierte Ultraschallwellen zu erzeugen, wobei die Stimulationseinrichtung optional zumindest einen Gehäuseabschnitt mit einer in Richtung des Kopfes orientierten Unterseite und einer in Richtung des Anwenders orientierten Oberseite umfasst. Die Anzeigevorrichtung ist vorzugsweise an der Oberseite vorgesehen oder in dieser integriert. Fokussierte Ultraschallstimulation (FUS) ist eine Technologie, bei der hochfrequente Schallwellen (Ultraschall) gezielt auf eine bestimmte Region im Körper, beispielsweise das Gehirn, gerichtet werden, um dort eine therapeutische Wirkung zu erzielen. Dies kann beispielsweise zur Behandlung von Tumoren oder zur Stimulation von Nervengewebe verwendet werden. Die fokussierte Ultraschallstimulation ist vorzugsweise eine fokussierte Stimulationstechnik, die insbesondere tiefere, subkortikale Ziele mit einer vorbestimmten Fokalität aktivieren kann. Die erfindungsgemäß angeordnete Anzeigevorrichtung bietet bei dieser Technologie tiefgreifende Vorteile, da durch FUS auch tiefere Gehirnstrukturen angesprochen werden sollen und dabei ein Kontakt zu dem Kopf und/oder ein Kontaktwinkel mit dem Kopf von zentraler Bedeutung ist/sind, um den Ultraschall an den Kopf zu koppeln und das weiter entfernte Ziel innerhalb des Gehirns zu erreichen. Erfindungsgemäß ist es nun möglich, durch den Schädel und vorzugsweise durch kortikale Strukturen zumindest virtuell hindurchzusehen, so dass auch tieferliegende Bereiche des Gehirns durch die Anzeigevorrichtung virtuell freigelegt und/oder angezeigt werden können, so dass der fokussierte Ultraschall auf einen derartigen Bereich exakter fokussiert werden kann. Hierdurch werden Behandlungsresultate nachhaltig verbessert. Die erfindungsgemäße Anzeigevorrichtung ist auch bei FUS-Systemen vorteilhaft, bei denen die Stimulationseinrichtung an einem Roboterführungsarm und/oder einem insbesondere stereotaktischen Rahmen und/oder an einer anderen mechanischen Vorrichtungen zumindest hinsichtlich einiger Bewegungsfreiheitsgrade geführt ist.

In einer bevorzugten Ausführungsform ist die Anzeigevorrichtung dazu ausgebildet, die mindestens eine Bildinformation mit mindestens einer Positionierungsinformation insbesondere überlagert anzuzeigen. Durch die mindestens eine Positionierungsinformation ist vorzugsweise eine Position und/oder Lage der Stimulationseinrichtung oder des chirurgischen Instruments relativ zu dem Körperteil des Patienten und/oder relativ zu der mindestens einen Bildinformation über den Körperteil des Patienten angegeben, um derart den Anwender bei der Positionierung der Stimulationseinrichtung zu unterstützen.

Die Position und/oder Lage (gleichbedeutend mit Orientierung) der Stimulationseinrichtung oder des chirurgischen Instruments relativ zu dem Körperteil, insbesondere dem Kopf, des Patienten kann beispielsweise durch Messung mit einem optischen, magnetischen oder elektromagnetischen System erfolgen. Insbesondere kann die Messung über Triangulation über zumindest zwei Perspektiven, also beispielsweise zumindest zwei Kameras, zwei magnetische Sender oder Detektoren oder zwei elektromagnetische Sender oder Detektoren erfolgen. Ferner kann die relative Position und/oder Lage über Messung einer Position und/oder Lage für sowohl den Körper, beispielsweise den Kopfe, des Patienten und für die Stimulationseinrichtung oder das chirurgische Instrument in einem gemeinsamen Koordinatensystem erfolgen, um daraus die relative Position und/oder Lage zu ermitteln. Hier können beispielsweise retroreflektive Marker, Leuchtquellen und/oder magnetische oder elektromagnetische Quellen, hier als Marker oder Sender zusammengefasst, am zu messenden Objekt oder Subjekt mit zumindest zwei Kameras, magnetischen oder elektromagnetischen Transducern, hier als Empfänger zusammengefasst, erfolgen. Ebenso lassen sich die Sender und Empfänger in diesen wie im Stand der Technik bekannt tauschen. Ferner lässt sich die relative Position und/oder Lage durch Messung der Position und/oder Lage eines ersten Elementes aus Stimulationseinrichtung oder chirurgischem Instrument und Körper, bspw. Kopf, von einem zweiten Element aus Stimulationseinrichtung oder chirurgischem Instrument und Körper, bspw. Kopf, messen. Hierzu ist ein Sender entsprechend an der Stimulationseinrichtung oder dem chirurgischen Instrument und ein Empfänger an dem betreffenden Körperteil, bspw. dem Kopf eines Patienten befestigt, wobei die paarweise Zuordnung auch wechseln lässt. Alternativ zu einer Messung von Positionen und/oder Lage von Markern lässt sich die Position und/oder Lage von Körper, beispielsweise dem Kopf, und/oder der Stimulationseinrichtung oder dem chirurgischen Instrument auch über maschinelles Sehen aus Kamerabildern mit anschließender Mustererkennung von anatomischen Merkmalen, beispielsweise Gesichtsmerkmalen, und/oder geometrischen Merkmalen der Stimulationseinrichtung messen.

In einer bevorzugten Ausführungsform umfasst die mindestens eine Positionierungsinformation Informationen über Annotationen im Raum und/oder Informationen über vorbestimmte, vorzugsweise für die Positionierung der Stimulationseinrichtung angegebene Wegpunkte im Raum und/oder positionsbezogene Textinformationen und/oder Informationen über eine positionsbezogene Aktivierungswolke der zu stimulierenden Neuronen und/oder neuronalen Schaltkreise und/oder positionsbezogene Informationen über bereits stimulierte Neuronen und/oder neuronale Schaltkreise. Optional ist die Anzeigevorrichtung also eingerichtet, auch Annotationen, wie Wegpunkte im Raum und/oder positionsbezogene Texte und/oder auch Aktivierungswolken aus einer funktionellen Bildgebung des Gehirns, beispielsweise mit einer Pseudofarbmarkierung, anzuzeigen.

Die Aktivierungswolke bezieht sich vorzugsweise auf eine Region bzw. einen Bereich des Gehirns und/oder des Nervengewebes, das durch Stimulation aktivierbar ist. Eine Größe und/oder Form der Aktivierungswolke hängt vorzugsweise von verschiedenen Faktoren ab, wie der Energie und/oder der Einstrahlung und/oder der Pulsdauer und/oder der Pulsform des Stimulierungssignals ab. Eine Kontrolle der Größe und/oder Form der Aktivierungswolke ist besonders bevorzugt, um die gewünschte Region des zu stimulierenden Bereiches möglichst exakt zu treffen und unerwünschte Auswirkungen auf andere Bereiche des Körpers zu vermeiden. Dies wird erfindungsgemäß durch die Anzeigevorrichtung erreicht.

Die mindestens eine positionsbezogene Information über bereits zuvor stimulierte Neuronen und/oder neuronale Schaltkreise kann beispielsweise mindestens einen Stimulationsort innerhalb des Gehirns und/oder eines sonstigen zu stimulierenden Körperteils des Patienten durch mindestens eine Markierung kennzeichnen. Eine solche Markierung kann beispielsweise ein optischer Marker, wie ein Kreuz oder ein Pfeil sein. Die Markierung kann vorzugsweise mindestens eine Orientierungsinformation umfassen.

In einer bevorzugten Ausführungsform umfasst die mindestens eine Positionierungsinformation positionsbezogene Informationen über einen Aktivierungsort und/oder ein Aktivierungsvolumen, die auf Basis einer räumlich in das Gehirn induzierten elektrischen Feldverteilung der Magnetspule ermittelbar sind. Bevorzugt verfügt die Anzeigevorrichtung also über einen Betriebsmodus, in dem ein zu erwartender Aktivierungsort und/oder ein zu erwartetes Aktivierungsvolumen aufgrund der räumlich induzierten elektrischen Feldverteilung oder aufgrund der räumlich induzierten Ultraschallverteilung anzeigt werden kann. Diese Feldverteilung und/oder die Ultraschallverteilung kann/können vorzugsweise unabhängig von einer Anatomie des Patienten sein. Es kann sich beispielsweise um die sogenannte Freiraum-Feldverteilung, auch Primärfeldverteilung genannt, handeln. Alternativ kann der zu erwartende Aktivierungsort und/oder das zu erwartete Aktivierungsvolumen in Abhängigkeit von anatomischen Merkmalen geschätzt werden. Hierbei kann auch eine Messdatenhistorie einbezogen werden.

Alternativ oder ergänzend umfasst die mindestens eine Positionierungsinformation Simulationsinformationen über einen Aktivierungsort und/oder ein Aktivierungsvolumen, die auf Basis eines Simulationsmodells zur Simulation der induzierten elektrischen Feldverteilung ermittelbar sind. Bevorzugt verfügt die Anzeigevorrichtung also über einen Betriebsmodus, in dem das zu erwartende induzierte elektrische Feld auf der Grundlage eines Simulationsmodells für elektromagnetische Felder ermittelt wird. Alternativ oder ergänzend ist ein Betriebsmodus vorgesehen, bei dem der zu erwartende Ort und/oder das zu erwartende Muster der neuronalen Aktivierung durch ein solches Simulationsmodell simuliert werden kann. Ein solches Simulationsmodell kann beispielsweise durch ein trainiertes neuronales Modell und/oder ein trainiertes neuronales Netz ausgebildet sein. Die Simulationsinformationen werden vorzugsweise in Echtzeit auf der Anzeigevorrichtung angezeigt.

In einer bevorzugten Ausführungsform umfasst die mindestens eine Bildinformation Informationen über eine anatomische Struktur und/oder Informationen über eine Gyrifikation einer weißen Masse des Gehirns und/oder Informationen funktioneller Gehirnbilder. Die mindestens eine Bildinformation kann vorzugsweise um zusätzliche Informationen, wie funktionelle Bilder und/oder Annotationen im Raum ergänzt sein. Gyrifikation bezieht sich vorzugsweise auf die Schichtung von weißem Hirngewebe. "Gyrifikation" ist der Prozess der Bildung von Falten oder Furchen in der Hirnrinde, die zu einer Vergrößerung der Oberfläche führen. Gyrifikation ist besonders ausgeprägt in den Bereichen des Gehirns, die für komplexe kognitive Funktionen wie Sprache, Gedächtnis und Planung verantwortlich sind.

Eine "weiße Masse" im Gehirn besteht insbesondere aus Myelinscheiden und Glia-Zellen, die Nervenfasern umhüllen und die Signalübertragung beschleunigen. Gyrifikation kann aufgrund von Schädigungen oder Erkrankungen des Gehirns beeinträchtigt werden, was zu einer Verminderung der kognitiven Funktionen führen kann.

In einer bevorzugten Ausführungsform ist die Anzeigeeinrichtung konzentrisch zu einem durch einen Fokuspunkt der Stimulationseinrichtung und/oder zu einer Hauptachse und/oder einer Hauptorientierung des chirurgischen Instruments verlaufenden Normalenvektor ausgerichtet. Der Normalenvektor verläuft vorzugsweise ausgehend von dem Fokuspunkt der Stimulationseinrichtung oder ausgehend von einem Aktuationszentrum des chirurgischen Instruments zum einem Anregungspunkt innerhalb des Köperteils, insbesondere des Gehirns und/oder des sonstigen zu stimulierenden Gewebes, oder zu einem Einsatzort des chirurgischen Instruments innerhalb des Körperteils. Der Normalenvektor verläuft vorzugsweise orthogonal zu der Unterseite der Stimulationseinrichtung oder des chirurgischen Instruments. Die Anzeigevorrichtung ist vorzugsweise derart ausgerichtet, dass sich der gedachte Normalenvektor mit ihrem geometrischen Mittelpunkt schneidet. Ein bevorzugter Bildschirm der Anzeigevorrichtung ist derart vorzugsweise über dem Fokuspunkt der Stimulationseinrichtung, beispielsweise der Magnetspule angeordnet.

In einer bevorzugten Ausführungsform ist auf einer Oberseite der Anzeigevorrichtung mindestens eine Positionsmarkierung aufgebracht. Alternativ oder ergänzend ist außerhalb der Anzeigevorrichtung auf der Oberseite der Stimulationseinrichtung eine korrespondierende und/oder anders ausgebildete Positionsmarkierung vorgesehen. Auf einer Oberfläche der Oberseite der Anzeigevorrichtung und/oder der Stimulationseinrichtung und/oder des chirurgischen Instruments kann/können vorzugsweise als die mindestens eine Positionsmarkierung ein Raster und/oder Gitter vorgesehen sein. Die Anzeigevorrichtung kann mit anderen Worten vorzugsweise ein festes (d. h. nicht veränderbares) Gitter umfassen, das eine Skala und/oder ein Fadenkreuz aufweist. Vorzugsweise definiert die Positionsmarkierung Abstände und/oder umfasst eine Markierung des geometrischen Mittelpunktes. Der Mittelpunkt der Anzeigevorrichtung kann auch virtuell auf dieser als Fixpunkt angezeigt sein, und definiert vorzugsweise auch den Fokuspunkt der Stimulationseinrichtung. Falls sich die mindestens eine Positionsmarkierung in einem gewissen Abstand von dem Bildschirm, betrachtet entlang des Normalenvektors, befindet, kann eine Maßnahme zur Kontrolle und/oder Korrektur der Parallaxe darin bestehen, die mindestens eine Positionsmarkierung zusätzlich auf dem Bildschirm darzustellen und den Anwender vorzugsweise anzuweisen, beide Positionsmarkierungen zu überlagern bzw. in Deckung zu bringen. In diesem Fall sollte die mindestens eine Positionsmarkierung 2 mm, bevorzugt > 5 mm, besonders bevorzugt > 10 mm über der Anzeigevorrichtung angeordnet sein. Die mindestens eine Positionsmarkierung kann auch ein Fadenkreuz umfassen. Die mindestens eine Positionsmarkierung kann in eine Deckschicht der Anzeigevorrichtung, die z. B. aus Glas oder Polymer gefertigt ist, gedruckt oder geätzt sein. Die mindestens eine Positionsmarkierung kann auch auf einer Folie aufgebracht sein, die vorzugsweise in einem Folienverbund der Anzeigevorrichtung angeordnet ist.

In einer bevorzugten Ausführungsform umfasst die Anzeigevorrichtung eine Parallaxeausgleichseinrichtung. Die Parallaxe ist vorzugsweise ein optisches Phänomen, bei dem ein scheinbarer Standort eines Objekts in Bezug auf seine Umgebung aufgrund der verschiedenen Beobachtungspunkte verändert erscheint. Die Parallaxeausgleichseinrichtung kann als eine insbesondere einschichtige Parallaxenkorrektur ausgebildet sein. Die Parallaxeausgleichseinrichtung kann dazu eingerichtet sein, eine Positionskorrektur zumindest in einer Richtung und/oder um eine Richtung für einen Anwender zu erleichtern. Die Parallaxeausgleichseinrichtung kann dazu ausgebildet sein, eine multidirektionale Positionskorrektur zu ermöglichen. Hierzu kann die Parallaxeausgleichseinrichtung bevorzugt eine Struktur, beispielsweise eine Wabenstruktur, umfassen, um mit dieser vorzugsweise Licht zu blockieren, das in einer der Richtungen, in denen die Stimulationseinrichtung oder das chirurgische Instrument parallaxefrei ausgerichtet werden soll, von der Norm abweichen. Die Wabenstruktur kann beispielsweise in der Aufsicht runde oder eckige Strukturen insbesondere aus lichtundurchlässigem Material umfassen, die eine vorbestimmte Höhe bis zur Filmdicke der Parallaxeausgleichseinrichtung, bspw. > 50 µm, vorzugsweise > 100 µm, besonders bevorzugt > 250 µm, weiter besonders bevorzugt > 500 µm oder gar > 1 mm, aufweist und vorzugsweise zahlreiche eng beieinander liegende lichtdurchlässige Aussparungen, beispielsweise sechs- oder mehreckige Löcher einer Wabenstruktur, aufweist, durch die das Licht die Parallaxeausgleichseinrichtung passieren kann, wenn der Einfallswinkel relativ zur Oberflächennormale nur eine geringe Winkelabweichung aufweist. Bei höheren Winkelabweichungen treffen Lichtstrahlen vorzugsweise auf das Material bzw. Wände der Strukturen aus lichtundurchlässigem Material. Vorzugsweise sind die Aussparungen dicht beieinanderliegend, beispielsweise weniger als eine Dicke der Parallaxeausgleichseinrichtung. Ferner weisen die Aussparungen vorzugsweise in alle Richtungen innerhalb der Ebene der Oberfläche ähnliche oder gar gleiche Dimensionen auf, wie sie beispielsweise Kreisen oder näherungsweise regelmäßigen Vielecken wie Hexagonen zu eigen sind.

In einer bevorzugten Ausführungsform umfasst die Anzeigevorrichtung eine Lichtlenkeinrichtung, die bevorzugt dazu eingerichtet ist, schräg von der Anzeigevorrichtung abgestrahltes Licht zumindest teilweise zu blockieren zu blockieren. Die Lichtlenkeinrichtung blockiert das abgestrahlte Licht vorzugsweise ab einem vorbestimmten Grenzwinkel gemessen von einer Normalen zu einer Oberfläche der Anzeigevorrichtung. Die Lichtlenkeinrichtung kann beispielsweise Licht, dass von der Anzeigevorrichtung ausgestrahlt wird, ab einem Winkel von 20° bevorzugt 15°, besonders bevorzugt 10° abweichend von der Normalen blockieren. Bevorzugt ist die Anzeigevorrichtung durch eine Lichtlenkeinrichtung parallaxenfrei und bringt den Anwender durch die Implementierung der Lichtlenkeinrichtung dazu, nahezu orthogonal auf eine Oberfläche der Anzeigevorrichtung bzw. in Richtung des Normalenvektors zu blicken. Hierzu kann die Lichtlenkeinrichtung optional mit einer Folie zur Steuerung der Lichtausrichtung ausgestattet sein. Solche Lichtlenkungsfolien umfassen vorzugsweise vertikale Strukturen, wie z. B. Lamellen, die das schräg von der Anzeigevorrichtung abgestrahlte Licht blockieren. Vorzugsweise ist die Lichtlenkeinrichtung nicht unmittelbar auf dem bevorzugten Bildschirm der Anzeigevorrichtung angeordnet, sondern minimal, von einer Oberfläche des Bildschirms parallel beabstandet angeordnet. "Minimal" bedeutet in diesem Zusammenhang vorzugsweise > 1 mm, bevorzugt > 2 mm, besonders bevorzugt > 5 mm, um vorzugsweise einen möglichen Blickwinkel für den Anwender weiter zu verringern. Wenn der Anwender in einem immer größeren Winkel zur Normalen, d.h. schräg auf den Bildschirm blickt, erscheint das für den Anwender ersichtliche Bild immer dunkler, so dass er dazu gebracht wird, wieder in paralleler Richtung des Normalenvektors auf den Bildschirm zu blicken. Der bevorzugte Abstand erlaubt den möglichen Blickwinkel unter dem das Bild noch nicht deutlich in der Helligkeit abgeschwächt ist und damit Parallaxefehler zu verringern.

Bevorzugt umfasst eine Lichtlenkeinrichtung gemäß der Erfindung eine feine Gitterstruktur aus nahe beieinander liegenden lichtundurchlässigen Stäben, deren Höhe kleiner oder gleich der Dicke der Lichtlenkeinrichtung ist, und Lichtstrahlen, die nicht senkrecht auf die Oberfläche der Lichtlenkeinrichtung treffen, ab einer bestimmten Abweichung von der Normalen in einer bestimmten Richtung nicht mehr geradlinig durch die Lichtlenkeinrichtung passieren lässt, ohne dass die Lichtstrahlen auf einen lichtundurchlässigen Stab des Gitters fallen, beispielsweise entweder das obere Ende eines Stabes oder das untere Ende eines anderen, aber nicht zwischen den beiden hindurch durch die Lichtlenkeinrichtung. Besonders bevorzugt umfasst die Ausführung zumindest zwei Lichtlenkeinrichtungen, die jeweils durch eine streifenartige Gitterstruktur zur Lichtlenkung oder Lichtwinkelbeschränkung auf die Oberflächennormale gebildet werden, wobei die Richtung der Stäbe der Gitterstruktur einen nicht vernachlässigbaren Winkel zueinander aufweisen, der vorzugsweise zumindest 45°, besonders bevorzugt etwa 90° aufweist. Lichtlenkeinrichtungen und damit die Gitterrichtung in Lichtlenkeinrichtungen sollten vorzugsweise parallel zur Bildschirmoberfläche verlaufen. Diese besonders bevorzugte Ausführungsform begrenzt Lichtstrahlen und damit den Parallaxefehler in allen beiden Winkeln eines Lichtstrahls zur Ebene der Lichtlenkeinrichtung bzw. des Bildschirmes oder zur Oberflächennormalen der Lichtlenkeinrichtung bzw. des Bildschirmes zu begrenzen.

In einer bevorzugten Ausführungsform umfasst die Anzeigevorrichtung eine Magnetfeldabschirmeinrichtung. Eine derartige Magnetfeldabschirmeinrichtung ist insbesondere dann bevorzugt, wenn die Stimulationseinrichtung eine Magnetspulenvorrichtung mit mindestens einer Magnetspule zur insbesondere transkraniellen Magnetstimulation (TMS) umfasst. TMS-Magnetspule senden vorzugsweise einen starken magnetischen Wechselfeldimpuls aus, der starke elektrische Felder und/oder Spannungen auch in nahe gelegene elektronische Geräte in der Peripherie der erfindungsgemäßen Vorrichtung induzieren kann. Diese Störquelle kann grundsätzlich auch den störungsfreien Betrieb der Anzeigevorrichtung beeinflussen. Zur Lösung des Problems ist die Magnetfeldabschirmeinrichtung bevorzugt. Die Magnetfeldabschirmeinrichtung umfasst beispielsweise ein magnetisches Material mit einer höheren magnetischen Leitfähigkeit als Luft, das dazu ausgebildet ist den magnetischen Fluss um die Anzeigevorrichtung herum zu leiten. Hierdurch wird zumindest weitestgehend verhindert, dass das elektromagnetische Feld in die Anzeigevorrichtung eindringt und dort eine parasitäre Spannungen induziert. Zudem konzentriert die Magnetfeldabschirmeinrichtung vorzugsweise den magnetischen Fluss auf die TMS-Magnetspule, und führt den magnetischen Fluss vorzugsweise näher an die TMS-Magnetspule heran, so dass weniger Energie zur Erzeugung eines Magnetfeldes einer vorbestimmten Stärke benötigt wird. Hierdurch wird als Nebeneffekt die Stimulationsspule auch energetisch effizienter. Das magnetische Material ist vorzugsweise Ferrit aufgrund seiner geringen elektrischen Leitfähigkeit. Durch den Einsatz von Ferrit können insbesondere hohe Energieverluste aufgrund von Wirbelströmen vermieden oder zumindest vermindert werden. Bevorzugt ist die Magnetfeldabschirmeinrichtung so groß wie die Anzeigevorrichtung dimensioniert. Besonders bevorzugt ist die Magnetfeldabschirmeinrichtung mindestens 10 bis 25 % größer als die Anzeigevorrichtung dimensioniert. Alternativ oder ergänzend sind andere magnetische Materialien, z. B. laminierte Stahlbleche, als Magnetfeldabschirmeinrichtung einsetzbar. Eine Dicke (betrachtet in Richtung der Normalen der Anzeigevorrichtung) ist mindestens so groß, dass 80 %, vorzugsweise >90 % des magnetischen Flusses der TMS-Magnetspule über den Fokuspunkt der Stimulationseinrichtung geleitet werden.

Zudem werden solche TMS-Magnetspule zumeist mit einer insbesondere abrupt bzw. schlagartig ansteigenden Hochspannung betrieben, was zu einer kapazitiven Kopplung mit nahe gelegenen Geräten und/oder Elektronik führen kann. Diese Störquelle kann grundsätzlich auch den störungsfreien Betrieb der Anzeigevorrichtung beeinflussen. Konventionelle kapazitive Entkopplungsansätze, wie sie beispielsweise in der Nachrichtentechnik verwendet werden, sind hier nicht anwendbar, da die Feldstärken um Größenordnungen höher sind als in der Nachrichtentechnik (bei TMS beispielsweise: ca. 1 - 2 T; 100 - 200 V/m. Zudem hat die TMS-Magnetspule eine vergleichsweise niedrige Impedanz, so dass eine Abschirmung zu einer massiven Erwärmung führen würde und sich negativ auf die durch die TMS-Magnetspule generierten magnetischen Felder auswirken würde, die jedoch gerade zur Stimulation benötigt werden und daher optimiert werden sollten. Zur Lösung umfasst in einer bevorzugten Ausführungsform die Anzeigevorrichtung eine elektrische Entkopplungseinrichtung, die dazu ausgebildet ist, eine kapazitive Kopplung zwischen der Stimulationseinrichtung und der Anzeigevorrichtung zumindest teilweise zu mindern. Besonders bevorzugt ist die Entkopplungseinrichtung dazu eingerichtet, kapazitive Kopplung zu verhindern oder zumindest zu vermeiden und dabei keine Wirbelströme zu erzeugen. Besonders bevorzugt ist die Entkopplungseinrichtung zwischen der Magnetfeldabschirmeinrichtung und einem Bildschirm der Anzeigevorrichtung angeordnet. Die optionale Entkopplungseinrichtung ist also idealerweise oberhalb des magnetischen Materials, d. h., zwischen der Magnetfeldabschirmeinrichtung und dem bevorzugten Bildschirm angeordnet. Die vorzugsweise Anordnung der Magnetfeldschirmeinrichtung zwischen Spule und Entkopplungseinrichtung verringert den magnetischen Fluss, der die Entkopplungseinrichtung senkrecht durchflutet, und damit auch zu erwartende induzierte Wirbelströme. So befindet sich der größte Teil des sich ändernden magnetischen Flusses in der Magnetfeldabschirmeinrichtung und es verbleibt nur ein niedriger magnetischer Fluss, der Wirbelströme verursachen könnte. Die Entkopplungseinrichtung umfasst beispielsweise eine elektrisch leitende Platte und/oder eine elektrisch leitende Kunststofffolie und/oder eine elektrisch leitende Lackschicht. Die Entkopplungseinrichtung umfasst vorzugsweise einen streifenartigen Aufbau mit zumindest weitgehend parallel angeordneten, elektrisch leitenden Elementen, die vorzugsweise über mindestens eine gemeinsame Busbar (Leiterschiene) bzw. Sammelleiter elektrisch leitend miteinander verbunden sind. Die Entkopplungseinrichtung ist vorzugsweise mit einem festen oder gepufferten elektrischen Potential verbunden, um derart elektrische Felder zu absorbieren. Vorzugsweise erlaubt die Entkopplungseinrichtung nur kleine kreis-, regelmäßige vieleckoder quadratförmige Pfade, die durchwegs in elektrisch leitfähigem Material verlaufen. Vorzugsweise liegen solche kreisförmige oder quadratförmige Pfade mit maximal 20 mm, vorzugsweise maximal 10 mm und besonders bevorzugt maximal 5 mm Durchmesser oder Kantenlänge vor, um Wirbelstromeinkopplungen und damit Energieverluste zu minimieren. Vorzugsweise ist ferner die größte durch einen Pfad umschriebene Fläche, wobei der Pfad ferner voll durch elektrisch leitfähiges Material verläuft, so klein wie möglich, beispielsweise nicht größer als 1 cm² und besonders bevorzugt nicht größer als 0.5 cm². Die Entkopplungseinrichtung ist vorzugsweise mit einem stabilen elektrischen Potential verbunden. Besonders vorzugsweise ist die Entkopplungseinrichtung mit Ground verbunden.

Erfindungsgemäß ist auch ein System zur nicht-invasiven Neurostimulation vorgeschlagen. Das System umfasst mindestens eine Leistungsquelle, eine Auswerte- und Recheneinrichtung und eine erfindungsgemäße Vorrichtung nach einer beliebigen Ausführungsform. Die Leistungsquelle ist vorzugsweise dazu eingerichtet, elektrische Pulse zu erzeugen, die mind. eine Spule durchfließen und induzierte elektrische Felder insbesondere auch mit einer schnellen Wiederholrate bzw. Periodendauer generieren. Die elektrischen Pulse erreichen vorzugsweise Stromscheitelwerte über 500 A, besonders bevorzugt über 1000 A, weiter bevorzugt über 3000 A und besonders bevorzugt über 5000 A. Die Spule weist vorzugsweise eine Induktivität im Bereich von 1 µH bis 20 mH, bevorzugt 5 µH bis 100 µH und besonders bevorzugt von 5 µH bis 50 µH auf. Die den Strom des elektrischen Pulses treibende Spannung erreicht vorzugsweise zumindest Scheitelwerte von 400 V, besonders bevorzugt zumindest 1000 V. Die magnetischen Pulse erreichen vorzugsweise zumindest eine Flussdichte mit Scheitelwert von zumindest 500 mT, besonders bevorzugt von zumindest 1 T. Die Pulsdauer liegt vorzugsweise im Bereich von 10 µs und 200 ms, für Motornerven in der Peripherie und von Neuronen im Gehirn ferner bevorzugt im Bereich von 50 µs und 2 ms. Die Pulse umfassen vorzugsweise eine variable Form und/oder Pulsdauer. Hierzu umfasst die Leistungsquelle vorzugsweise eine hierzu eingerichtete Leistungselektronik.

Die beschriebenen Ausgestaltungen und Weiterbildungen lassen sich beliebig miteinander kombinieren.

Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung.

### Kurze Beschreibung der Zeichnungen

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung.

Andere Ausführungsformen und eine Vielzahl der genannten Vorteile ergeben sich im Hinblick auf die Zeichnungen. Die dargestellten Elemente der Zeichnungen sind nicht notwendigerweise maßstabsgetreu zueinander gezeigt.

Es zeigen:
- Fig. 1: eine schematische Ansicht eines Ausführungsbeispiels eines Systems zur nicht-invasiven Neurostimulation;
- Fig. 2: eine schematische Explosionsansicht eines ersten Ausführungsbeispiels einer Vorrichtung zur nicht-invasiven Neurostimulation;
- **Fig.** 3: eine schematische Explosionsansicht eines zweiten Ausführungsbeispiels einer Vorrichtung zur nicht-invasiven Neurostimulation;
- Fig. 4: eine schematische Explosionsansicht eines dritten Ausführungsbeispiels einer Vorrichtung zur nicht-invasiven Neurostimulation; und
- Fig. 5: eine schematische Explosionsansicht eines vierten Ausführungsbeispiels einer Vorrichtung zur nicht-invasiven Neurostimulation.

In den Figuren der Zeichnungen bezeichnen gleiche Bezugszeichen gleiche oder funktionsgleiche Elemente, Bauteile oder Komponenten, soweit nichts Gegenteiliges angegeben ist.

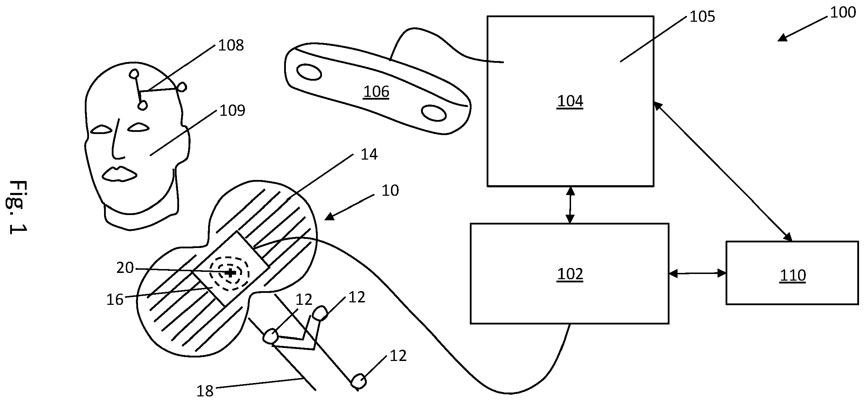

Figur 1 zeigt eine schematische Ansicht eines Ausführungsbeispiels eines Systems 100 zur nicht-invasiven Neurostimulation. Das System umfasst eine Leistungsquelle 102, eine Auswerte- und Recheneinrichtung 104 und eine Vorrichtung 10 zur nicht-invasiven Neurostimulation. Ferner umfasst das System gemäß der gezeigten Ausführung bevorzugt eine Positions- und/oder Lageerkennungseinrichtung 105 mit mindestens einer Stereokamera 106. Die Positions- und/oder Lageerkennungseinrichtung 105 ist vorliegend Teil der Auswerte- und Recheneinrichtung 104, kann jedoch auch getrennt von dieser ausgeführt sein. Die Positions- und/oder Lageerkennungseinrichtung 105 ist vorzugsweise dazu eingerichtet, eine Position und/oder Lage eines Kopfes und/oder eines sonstigen Körperteils eines Patienten im Raum zu erfassen. Hierzu werden vorzugsweise Positions- und/oder Lageinformationen von optischen Markern 108, die an dem Kopf 109 und/oder dem Körperteil des Patienten angeordnet sind, über die Stereokamera 106 verfolgt, und über trigonometrische Bildauswertung die zugehörige Position und/oder Lage im Koordinatensystem der Stereokamera ermittelt. Durch Koordinatentransformation können die Koordinaten vorzugsweise in ein räumliches Hauptbezugssystem umgerechnet werden. Die Positions- und/oder Lageerkennungseinrichtung 105 ist vorzugsweise dazu eingerichtet, eine Position und/oder Lage der Vorrichtung im Raum zu erfassen. Hierzu umfasst die Vorrichtung 10 vorzugsweise optische Marker 12. Zur eindeutigen Bestimmung der Position und/oder Lage im Raum sind vorzugsweise jeweils mindestens drei optische Marker an dem Kopf des Patienten und/oder an der Vorrichtung 10 angeordnet.

Aus der ermittelten Position und/oder Lage des Kopfes 109 oder des sonstigen Körperteils des Patienten sowie der Position und/oder Lage der Vorrichtung im Raum kann eine Relativposition zwischen dem Kopf 109 und der Vorrichtung 10 errechnet werden. Diese Berechnung erfolgt vorzugsweise durch die Auswerte- und Recheneinrichtung 104.

Die Vorrichtung 10 umfasst eine Stimulationseinrichtung 14, die ausgebildet ist, relativ zu dem Kopf 109 des Patienten positionierbar zu sein. Die Stimulationseinrichtung 14 ist dazu eingerichtet, mindestens ein Stimulationssignal zu erzeugen, durch das mindestens ein Neuron und/oder einen neuronalen Schaltkreis im Gehirn des Patienten und/oder eine Nervenzelle in einem sonstigen Körperteil anregbar ist. Die Stimulationseinrichtung 14 umfasst eine Anzeigevorrichtung 16 in Form eines Displays. Die Anzeigeeinrichtung ist dazu eingerichtet, mindestens eine Bildinformation, insbesondere eine Gehirnscan-Information und/oder eine generische Gehirndateninformation, über ein Gehirn des Patienten vorzugsweise zusammen mit Positionsbezogenen Informationen über die Position und/oder Lage der Vorrichtung 10 relativ zu dem Kopf 109 des Patienten anzuzeigen. Hierdurch wird der Anwender bei der Positionierung der Stimulationseinrichtung 14 unterstützt. Die Stimulationseinrichtung 14 umfasst vorliegend eine Griffabschnitt 18, durch den die Stimulationseinrichtung 14 von dem Anwender manuell angegriffen und dadurch im Raum positioniert werden kann.

Bevorzugt umfasst das System eine Berechnungseinrichtung 110, die dazu eingerichtet ist, eine Echtzeitkalkulation des Stimulationssignals und/oder einer durch das Stimulationssignal erzeugten Stimulation auszugeben, um diese insbesondere im Form der mindestens eine Bildinformation auf der Anzeigevorrichtung 16 gemeinsam mit anderen Informationen anzuzeigen, so dass die Positionierung der Vorrichtung 10 relativ zu dem Kopf 109 des Patienten für den Anwender nachhaltig vereinfacht wird. Die Komponenten 102, 104 und 110 sind vorzugsweise dazu ausgebildet jeweils untereinander zu kommunizieren, wie dies durch eine Doppelpfeildarstellung angezeigt ist. Vorzugsweise umfasst eine Anzeige der mindestens einen Bildinformation eine Projektion in eine Vorrichtungsperspektive, die insbesondere durch eine Sichtlinie senkrecht zur einer Vorrichtungsnormalen (in Fig. 1 in Blattebene hineinverlaufend) und durch einen Fokuspunkt 20 der Vorrichtung bestimmt ist. Die mindestens eine Bildinformation wird vorzugsweise über ein oder mehrere Kabel oder kabellos an die Anzeigevorrichtung 16 übertragen und dort zur Positionierungshilfe angezeigt.

Die Berechnungseinrichtung berechnet vorzugsweise eine Darstellung von dreidimensionalen Anatomiedaten des Patienten oder der Patientin oder eines generischen Anatomiedatensatzes von zumindest einer Struktur beispielsweise des Gehirnes und verwendet hierzu die Blickposition und Blickrichtung des/der Bedieners/Bedienerin. Bevorzugterweise wird hierzu nicht die Kopfposition und Blickrichtung des/der Bedieners/Bedienerin gemessen, sondern mithilfe einer Parallaxefehler reduzierenden Einrichtung, beispielsweise Parallaxeausgleichseinrichtungen oder Lichtlenkeinrichtungen, auf etwa die Oberflächennormale des Stimulationsaktuators, beispielsweise einer Stimulationsspule oder Magnetspule oder einem FUS-Transducers, bzw. des Bildschirmes verringert und von der Berechnungseinrichtung angenommen. Entsprechend kann die Berechnungseinrichtung beispielsweise ein bekanntes 3D-Projektionsverfahren auf die Bildschirmebene oder bekannte Bibliotheken wie OpenGL oder DirectX einsetzen und die Kameraposition auf die zu erwartende Augenposition und -richtung auf der Normalen legen. Entsprechend kann die Berechnungseinrichtung die Anatomiedaten relativ mit der vorzugsweise optisch oder elektromagnetisch gemessenen Kopfposition und Kopforientierung des Patienten/der Patientin im virtuellen 3D-Raum positionieren und dynamisch verschieben und drehen, während die Kameraposition auf einen bestimmten Punkt auf der Normalen der Bildschirmoberfläche gelegt wird und die Kameraachse der genannten Oberflächennormale folgt. Mit jeder Änderung der vorzugsweise optisch oder elektromagnetisch gemessenen Position und Orientierung des Stimulationsaktuators, beispielsweise Spule, oder des Bildschirmes werden ebenso Kameraposition und Kameraorientierung in der Bilderzeugung bzw. Projektion von 3D auf 2D aktualisiert. Entsprechend erscheinen aus der Blickrichtung des Bedieners/der Bedienerin die auf dem Bildschirm dargestellten 3D-Anatomieaten vorzugsweise perspektivisch an der zu erwartenden Stelle relativ zum real unter dem Bildschirm bzw. dem Stimulationsaktuator liegenden Kopf. Alternativ zur separaten Positionierung und Orientierung der anatomischen Strukturen der gemessenen Position und Orientierung des Kopfes des Patienten/der Patientin und der Kamera in der Projektionsberechnung mit der Position und Orientierung des Bildschirmes bzw. des Stimulationsaktuators kann auch eine der beiden Positionen und Orientierung (von anatomischer Struktur oder der Kamera) in der Berechnung konstant gehalten werden und die jeweils andere Position und Orientierung (von Kamera oder anatomischer Struktur) mit jeder Änderung der relativen Position und Orientierung des Kopfes relativ zur Position und Orientierung des Bildschirmes bzw. Stimulationsaktuators, bspw. Spule oder FUS-Transducer, aktualisiert und entsprechend eingestellt werden, bevor das neue 2D-Projektionsbild berechnet und der Bildschirm aktualisiert wird.

Erfindungsgemäß ist ein computerimplementiertes Verfahren zur Berechnung von Bildinformationen und/oder einer Darstellung von insbesondere dreidimensionalen Anatomiedaten eines Patienten und/oder eines generischen Anatomiedatensatzes von zumindest einer Struktur, beispielsweise eines Gehirnes, vorgeschlagen. Zur Berechnung wird/werden vorzugsweise eine Blickposition und/oder eine Blickrichtung eines Anwenders verwendet. Bevorzugterweise wird hierzu nicht eine Kopfposition und/oder eine Blickrichtung des Anwenders gemessen, sondern mithilfe einer Parallaxefehler reduzierenden Einrichtung, beispielsweise einer Parallaxeausgleichseinrichtung und/oder einer Lichtlenkeinrichtung, in Bezug auf eine Oberflächennormale der der Stimulationsvorrichtung, beispielsweise einer Stimulationsspule oder Magnetspule oder einem FUS-Transducers, bzw. der Anzeigevorrichtung verringert. Der verringerte Blickwinkel bzw. das verringerte Blickfeld wird vorzugsweise zur Berechnung angenommen. Entsprechend kann durch das erfindungsgemäße Verfahren beispielsweise ein bekanntes 3D-Projektionsverfahren auf die Bildschirmebene der Anzeigeeinrichtung oder bekannte Bibliotheken wie OpenGL oder DirectX einsetzen und/oder eine Kameraposition auf die zu erwartende Augenposition und/oder -richtung auf der Normalen legen. Entsprechend können die Anatomiedaten relativ mit der vorzugsweise optisch oder elektromagnetisch gemessenen Kopfposition und/oder Kopforientierung des Patienten im virtuellen 3D-Raum positionieren und/oder dynamisch verschoben und/oder gedreht werden, insbesondere während die Kameraposition auf einen bestimmten Punkt auf der Normalen der Anzeigeoberfläche gelegt wird und eine Kameraachse der genannten Oberflächennormale folgt. Mit jeder Änderung der vorzugsweise optisch und/oder elektromagnetisch gemessenen Position und/oder Orientierung der Vorrichtung und/oder der Anzeigeeinrichtung werden die Kameraposition und/oder Kameraorientierung in der Bilderzeugung bzw. Projektion von 3D auf 2D aktualisiert. Entsprechend erscheinen aus der Blickrichtung des Anwenders die auf der Anzeigeeinrichtung dargestellten 3D-Anatomieaten vorzugsweise perspektivisch an der zu erwartenden Stelle relativ zum real unter dem Bildschirm bzw. der Vorrichtung befindlichen Kopf. Alternativ zur separaten Positionierung und/oder Orientierung der anatomischen Strukturen der gemessenen Position und/oder Orientierung des Kopfes des Patienten und/oder der Kamera in der Projektionsberechnung mit der Position und Orientierung des Bildschirmes bzw. des Stimulationsaktuators kann auch eine der beiden Positionen und Orientierung (von anatomischer Struktur oder der Kamera) in der Berechnung konstant gehalten werden und die jeweils andere Position und Orientierung (von Kamera oder anatomischer Struktur) mit jeder Änderung der relativen Position und Orientierung des Kopfes relativ zur Position und/oder Orientierung der Anzeigeeinrichtung bzw. der Vorrichtung aktualisiert und/oder entsprechend eingestellt werden, insbesondere bevor das neue 2D-Projektionsbild berechnet und auf der Anzeigeeinrichtung in Form der Bildinformation(en) aktualisiert wird bzw. dargestellt wird.

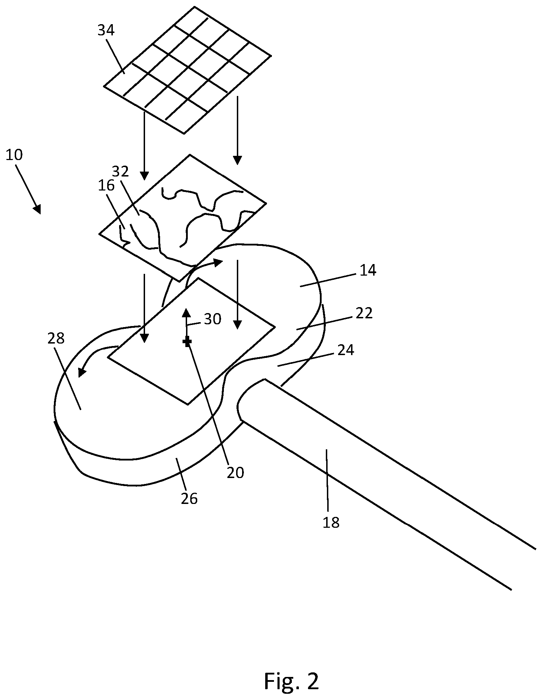

In den Figuren 2 bis 5 sind verschiedene Ausführungen der erfindungsgemäßen Vorrichtung 10 jeweils in einer zumindest teilweisen Explosionsdarstellung gezeigt. Die Stimulationseinrichtung 12 umfasst jeweils eine Magnetspulenvorrichtung 22 mit mindestens einer Magnetspule 24 zur insbesondere transkraniellen Magnetstimulation. Die Magnetspulenvorrichtung 22 ist ausgebildet, dass mindestens eine Stimulationssignal als elektromagnetische Wellen in Form eines Magnetfeldes zu erzeugen. Die Magnetspulenvorrichtung 22 umfasst vorzugsweise zumindest einen Gehäuseabschnitt 26 mit einer in Richtung des Kopfes 109 orientierten Unterseite (nicht gezeigt) und einer in Richtung des Anwenders orientierten, der Unterseite gegenüberliegenden Oberseite 28. Die Anzeigevorrichtung 16 ist an der Oberseite 28 vorgesehen. Die Anzeigeeinrichtung 16 ist relativ zu der Stimulationseinrichtung 12 konzentrisch zu einem durch einen Fokuspunkt 20 der Stimulationseinrichtung 12 verlaufenden, rein imaginären Normalenvektor 30 ausgerichtet ist. Die Anzeigevorrichtung 16 umfasst gemäß Figur 2 eine auf einer Oberseite 32 der Anzeigevorrichtung 16 angeordnete oder aufgebrachte Positionsmarkierung 34. Die Positionsmarkierung 34 umfasst vorliegend ein Gitter und ist auf einer ansonsten transparenten Folie aufgedruckt und auf die Oberseite 32 der Anzeigevorrichtung 16 auflaminiert.

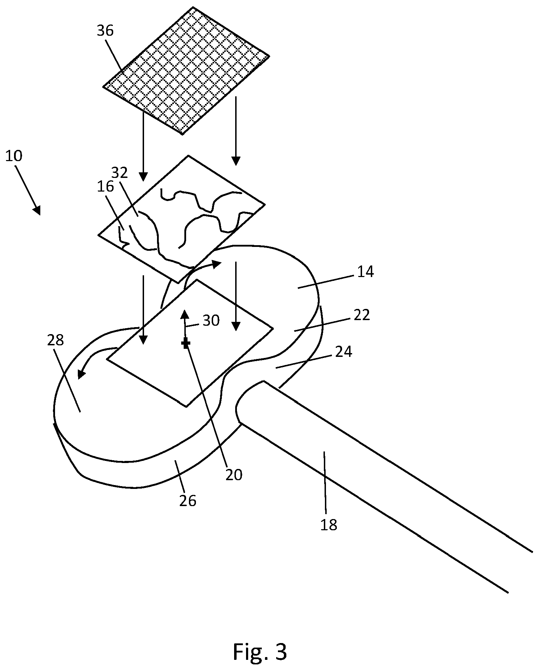

Die Vorrichtung 10 gemäß Figur 3 entspricht im Wesentlichen der Ausführung aus Figur 2, wobei die Vorrichtung 10 anstelle der Positionsmarkierung 34 eine Lichtlenkeinrichtung 36 umfasst, die dazu eingerichtet ist, schräg von der Anzeigevorrichtung 16 abgestrahltes Licht zu blockieren. Die Lichtlenkeinrichtung umfasst vorzugsweise eine wabenartige Struktur. Die Lichtlenkeinrichtung 36 ist auf der Oberseite 32 der Anzeigevorrichtung 16 oder von dieser etwas, beispielsweise 1 bis 10 mm, beabstandet angeordnet.

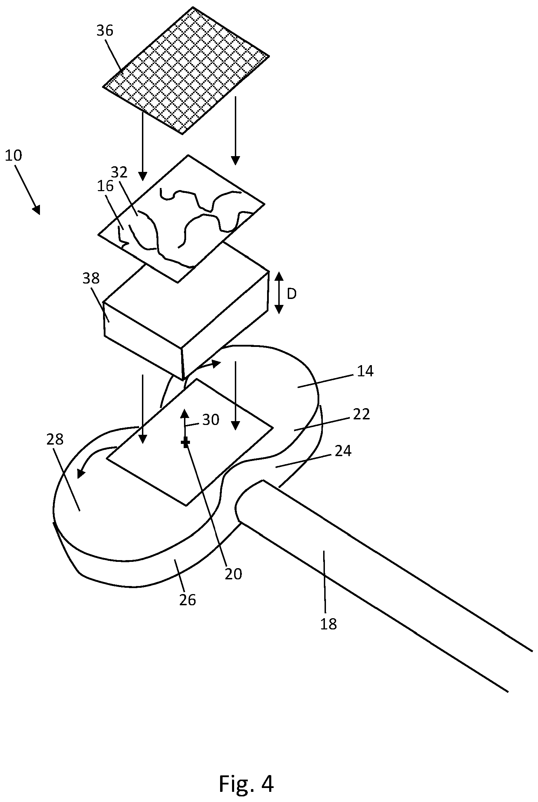

Gemäß Figur 4 umfasst die Anzeigevorrichtung 16 eine Magnetfeldabschirmeinrichtung 38, die als Block mit einer vorbestimmten Dicke D dargestellt ist. Die Magnetfeldabschirmeinrichtung 38 ist vorzugsweise zwischen der Magnetspulenvorrichtung 22 und der Anzeigevorrichtung 16 angeordnet.

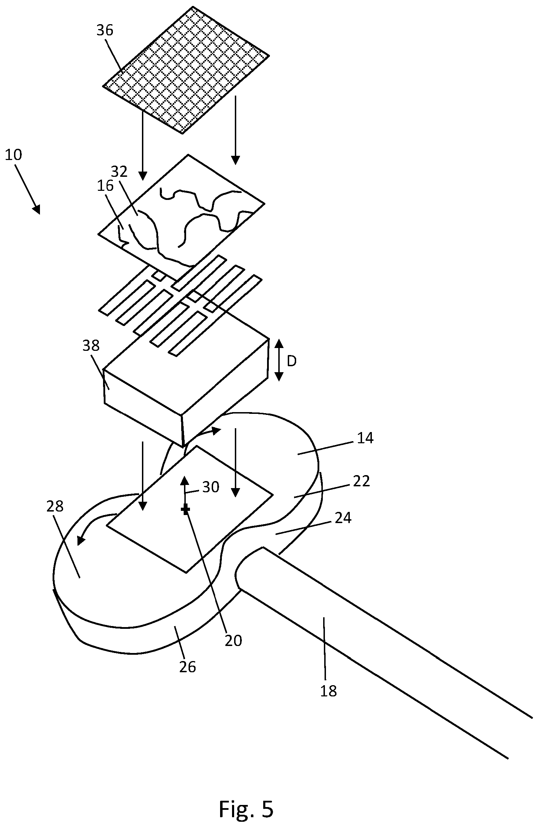

Gemäß Figur 5 umfasst die Anzeigevorrichtung 16 eine Entkopplungseinrichtung 40, die dazu ausgebildet ist, eine kapazitive Kopplung zwischen der Stimulationseinrichtung 12 und der Anzeigevorrichtung 16 zumindest teilweise zu mindern. Die Entkopplungseinrichtung 40 ist zwischen der Magnetfeldabschirmeinrichtung 38 und einem Bildschirm der Anzeigevorrichtung 16 angeordnet.

Die für die Vorrichtung 10 zur nicht-invasiven Neurostimulation gemachten Ausführen gelten in gleicher oder zumindest schematisch ähnlicher Weise für eine chirurgische Vorrichtung, die vorliegend jedoch nicht von dem Schutzumfang der vorliegenden Erfindung umfasst ist, ohne für eine derartige chirurgische Vorrichtung nochmals redundant genannt zu werden.

## Patentansprüche

1. Vorrichtung (10) zur nicht-invasiven Neurostimulation, umfassend eine Stimulationseinrichtung (12), die mindestens eine Anzeigevorrichtung (16) aufweist, wobei die Anzeigevorrichtung (16) an der Stimulationseinrichtung (12) angeordnet oder in dieser integriert ist, **dadurch gekennzeichnet, dass** die Anzeigevorrichtung (16) dazu eingerichtet ist, mindestens eine Bildinformation, umfassend eine anatomische und/oder eine generische anatomische und/oder funktionale und/oder physiologische Information über ein Gehirn eines Patienten, anzuzeigen, um derart einen Anwender bei der Positionierung der Stimulationseinrichtung (12) zu unterstützen.

2. Vorrichtung nach Anspruch 1, wobei die Stimulationseinrichtung (12) ausgebildet ist, relativ zu einem Körperteil, insbesondere einem Kopf (109) des Patienten positionierbar zu sein, wobei die Stimulationseinrichtung (12) vorzugsweise ausgebildet ist, mindestens ein Stimulationssignal zu erzeugen, durch das mindestens ein Neuron und/oder ein neuronaler Schaltkreis anregbar ist.

3. Vorrichtung nach Anspruch 1 oder 2, wobei die Stimulationseinrichtung (12) eine Magnetspulenvorrichtung (22) mit mindestens einer Magnetspule (24) zur insbesondere transkraniellen Magnetstimulation umfasst, die ausgebildet ist, das mindestens eine Stimulationssignal als elektromagnetische Wellen zu erzeugen, wobei die Magnetspulenvorrichtung (22) zumindest einen Gehäuseabschnitt mit einer in Richtung des Kopfes (109) orientierten Unterseite und einer in Richtung des Anwenders orientierten Oberseite (28) umfasst, wobei die Anzeigevorrichtung (16) an der Oberseite (28) vorgesehen ist.

4. Vorrichtung nach Anspruch 1 oder 2, wobei die Stimulationseinrichtung (12) dazu ausgebildet ist, das mindestens eine Stimulationssignal als insbesondere fokussierte Ultraschallwellen zu erzeugen, wobei die Stimulationseinrichtung (12) zumindest einen Gehäuseabschnitt mit einer in Richtung des Kopfes (109) orientierten Unterseite und einer in Richtung des Anwenders orientierten Oberseite (28) umfasst, wobei die Anzeigevorrichtung (16) an der Oberseite (28) vorgesehen ist.

5. Vorrichtung nach einem der vorstehenden Ansprüche, wobei die Anzeigevorrichtung (16) dazu ausgebildet ist, die mindestens eine Bildinformation mit mindestens einer Positionierungsinformation insbesondere überlagert anzuzeigen, wobei durch die mindestens eine Positionierungsinformation eine Position und/oder Lage der Stimulationseinrichtung (12) relativ zu dem Körperteil des Patienten und/oder relativ zu der mindestens einen Bildinformation über den Körperteil des Patienten angegeben ist, um derart den Anwender bei der Positionierung der Stimulationseinrichtung (12) zu unterstützen.

6. Vorrichtung nach Anspruch 5, wobei die mindestens eine Positionierungsinformation Informationen über Annotationen im Raum und/oder Informationen über vorbestimmte, vorzugsweise für die Positionierung der Stimulationseinrichtung (12) angegebene Wegpunkte im Raum und/oder positionsbezogene Textinformationen und/oder Informationen über eine positionsbezogene Aktivierungswolke der zu stimulierenden Neuronen und/oder neuronalen Schaltkreise und/oder positionsbezogene Informationen über bereits stimulierte Neuronen und/oder neuronale Schaltkreise umfasst.

7. Vorrichtung nach den Ansprüchen 3 und 6, wobei die mindestens eine Positionierungsinformation positionsbezogene Informationen über einen Aktivierungsort und/oder ein Aktivierungsvolumen umfasst, wobei die Vorrichtung ausgebildet ist, die positionsbezogene Informationen die auf Basis einer räumlich in das Gehirn induzierten elektrischen Feldverteilung der Magnetspule zu ermitteln; und/oder wobei die mindestens eine Positionierungsinformation Simulationsinformationen über einen Aktivierungsort und/oder ein Aktivierungsvolumen umfasst, wobei die Vorrichtung ausgebildet ist, die Simulationsinformationen auf Basis eines Simulationsmodells zur Simulation der induzierten elektrischen Feldverteilung zu ermitteln.

8. Vorrichtung nach einem der vorstehenden Ansprüche, wobei die mindestens eine Bildinformation Informationen über eine anatomische Struktur und/oder Informationen über eine Gyrifikation einer weißen Masse des Gehirns und/oder Informationen funktioneller Gehirnbilder umfasst.

9. Vorrichtung nach einem der vorstehenden Ansprüche, wobei die Anzeigeeinrichtung (16) relativ zu der Stimulationseinrichtung (12) konzentrisch zu einem durch einen Fokuspunkt (20) der Stimulationseinrichtung (12) verlaufenden Normalenvektor ausgerichtet ist.

10. Vorrichtung nach Anspruch 3 oder 4, wobei auf einer Oberseite (32) der Anzeigevorrichtung (16) mindestens eine Positionsmarkierung (34) angeordnet ist.

11. Vorrichtung nach einem der vorstehenden Ansprüche, wobei die Anzeigevorrichtung (16) mindestens eine Lichtlenkeinrichtung (36) umfasst, die dazu eingerichtet ist, schräg von der Anzeigevorrichtung abgestrahltes Licht zu blockieren.

12. Vorrichtung nach einem der vorstehenden Ansprüche, wobei die Anzeigevorrichtung (16) eine Parallaxeausgleichseinrichtung und/oder eine Magnetfeldabschirmeinrichtung (38) umfasst.

13. Vorrichtung nach einem der vorstehenden Ansprüche, wobei die Anzeigevorrichtung (16) eine Entkopplungseinrichtung (40) umfasst, die dazu ausgebildet ist, eine kapazitive Kopplung zwischen der Stimulationseinrichtung (12) und der Anzeigevorrichtung (16) zumindest teilweise zu mindern.

14. Vorrichtung nach Anspruch 12 und 13, wobei die Entkopplungseinrichtung (40) zwischen der Magnetfeldabschirmeinrichtung (38) und einem Bildschirm der Anzeigevorrichtung (16) angeordnet ist.

15. Vorrichtung nach Anspruch 11, wobei die Anzeigevorrichtung (16) mindestens eine erste und eine zweite Lichtlenkeinrichtung (36) umfasst, die dazu eingerichtet sind, schräg von der Anzeigevorrichtung abgestrahltes Licht zu blockieren und die erste Lichtlenkeinrichtung (36) über der zweiten Lichtlenkeinrichtung (36) angeordnet ist und gegenüber der zweiten Lichtlenkeinrichtung um 90° ± 20° relativ zu der Oberflächennormale der zweiten Lichtlenkeinrichtung (36) gedreht ist.

16. System zur nicht-invasiven Neurostimulation, umfassend mindestens eine Leistungsquelle (102), eine Auswerte- und Recheneinrichtung (104), und eine Vorrichtung (10) nach einem der vorstehenden Ansprüche.

## Claims

1. A device (10) for non-invasive neurostimulation, the device (10) comprising a stimulation unit (12) having at least one display device (16), the display device (16) being disposed on or integrated in the stimulation unit (12), **characterized in that** the display device (16) is configured to display at least one image information comprising an anatomical and/or a generic anatomical and/or functional and/or physiological information regarding a brain of a patient in order to thereby assist a user in positioning the stimulation unit (12).

2. The device according to claim 1, wherein the stimulation unit (12) is configured to be positioned relative to a body part, in particular a head (109) of the patient, the stimulation unit (12) being preferably configured to produce at least one stimulation signal capable of exciting at least one neuron and/or a neural circuit.

3. The device according to claim 1 or 2, wherein the stimulation unit (12) comprises a magnetic coil device (22) having at least one magnetic coil (24) for magnetic stimulation, in particular transcranial magnetic stimulation, the magnetic coil device (22) being configured to produce the at least one stimulation signal in the form of electromagnetic waves, the magnetic coil device (22) comprising at least one housing portion having a lower side oriented toward the head (109) and an upper side (28) oriented toward the user, the display device (16) being provided on the upper side (28).

4. The device according to claim 1 or 2, wherein the stimulation unit (12) is configured to produce the at least one stimulation signal in the form of ultrasonic waves, in particular focused ultrasonic waves, the stimulation unit (12) comprising at least one housing portion having a lower side oriented toward the head (109) and an upper side (28) oriented toward the user, the display device (16) being provided on the upper side (28).

5. The device according to any one of the preceding claims, wherein the display device (16) is configured to display the at least one image information with at least one positioning information, in particular in overlay, the at least one positioning information indicating a position and/or orientation of the stimulation unit (12) relative to the body part of the patient and/or relative to the at least one image information regarding the body part of the patient in order to thereby assist the user in positioning the stimulation unit (12).

6. The device according to claim 5, wherein the at least one positioning information comprises information regarding annotations in space and/or information regarding predetermined waypoints in space, which are preferably specified for positioning the stimulation unit (12), and/or position-related text information, and/or information regarding a position-related activation cloud of the neurons and/or neural circuits to be stimulated, and/or position-related information regarding neurons and/or neural circuits that have already been stimulated.

7. The device according to claims 3 and 6, wherein the at least one positioning information comprises position-related information regarding an activation site and/or an activation volume, the device being configured to determine the position-related information based on an electric field distribution of the magnetic coil spatially induced in the brain; and/or wherein the at least one positioning information comprises simulation information regarding an activation site and/or an activation volume, the device being configured to determine the simulation information based on a simulation model for simulating the induced electric field distribution.

8. The device according to any one of the preceding claims, wherein the at least one image information comprises information regarding an anatomical structure, and/or information regarding a gyrification of white matter of the brain, and/or information from functional brain images.

9. The device according to any one of the preceding claims, wherein the display device (16) is aligned concentrically with a normal vector passing through a focal point (20) of the stimulation unit (12) relative to the stimulation unit (12).

10. The device according to claim 3 or 4, wherein at least one position marker (34) is disposed on an upper surface (32) of the display device (16).

11. The device according to any one of the preceding claims, wherein the display device (16) comprises at least one light-guiding member (36) configured to block light emitted obliquely from the display device.

12. The device according to any one of the preceding claims, wherein the display device (16) comprises a parallax compensation member and/or a magnetic field shielding member (38).

13. The device according to any one of the preceding claims, wherein the display device (16) comprises a decoupling member (40) configured to at least partially reduce capacitive coupling between the stimulation unit (12) and the display device (16).

14. The device according to claims 12 and 13, wherein the decoupling member (40) is disposed between the magnetic field shielding member (38) and a screen of the display device (16).

15. The device according to claim 11, wherein the display device (16) comprises at least a first and a second light-guiding member (36) configured to block light emitted obliquely from the display device, and the first light-guiding member (36) is disposed above the second light-guiding member (36) and is rotated relative to the second light-guiding member by 90° ± 20° with respect to the surface normal of the second light-guiding member (36).

16. A system for non-invasive neurostimulation, the system comprising at least one power source (102), an evaluation and computing unit (104), and a device (10) according to any one of the preceding claims.

## Revendications

1. Dispositif (10) de neurostimulation non invasive, le dispositif (10) comprenant une unité de stimulation (12) ayant au moins un dispositif d'affichage (16), le dispositif d'affichage (16) étant disposé sur l'unité de stimulation (12) ou intégré dans celle-ci, **caractérisé en ce que** le dispositif d'affichage (16) est configuré pour afficher au moins une information d'image comprenant une information anatomique et/ou une information anatomique générique et/ou une information fonctionnelle et/ou une information physiologique concernant un cerveau d'un patient afin d'aider ainsi un utilisateur à positionner l'unité de stimulation (12).

2. Dispositif selon la revendication 1, dans lequel l'unité de stimulation (12) est configurée pour être positionnée par rapport à une partie de corps, notamment une tête (109) du patient, l'unité de stimulation (12) étant configurée de préférence pour produire au moins un signal de stimulation permettant d'exciter au moins un neurone et/ou un circuit neuronal.

3. Dispositif selon la revendication 1 ou 2, dans lequel l'unité de stimulation (12) comprend un dispositif à bobines magnétiques (22) ayant au moins une bobine magnétique (24) pour la stimulation magnétique, notamment la stimulation magnétique transcrânienne, le dispositif à bobines magnétiques (22) étant configuré pour produire l'au moins un signal de stimulation sous la forme d'ondes électromagnétiques, le dispositif à bobines magnétiques (22) comprenant au moins une partie de boîtier ayant une face inférieure orientée vers la tête (109) et une face supérieure (28) orientée vers l'utilisateur, le dispositif d'affichage (16) étant prévu sur la face supérieure (28).

4. Dispositif selon la revendication 1 ou 2, dans lequel l'unité de stimulation (12) est configurée pour produire l'au moins un signal de stimulation sous la forme d'ondes ultrasonores, notamment sous la forme d'ondes ultrasonores focalisées, l'unité de stimulation (12) comprenant au moins une partie de boîtier ayant une face inférieure orientée vers la tête (109) et une face supérieure (28) orientée vers l'utilisateur, le dispositif d'affichage (16) étant prévu sur la face supérieure (28).

5. Dispositif selon l'une quelconque des revendications précédentes, dans lequel the dispositif d'affichage (16) est configuré pour afficher l'au moins une information d'image avec au moins une information de positionnement, notamment en chevauchement, l'au moins une information de positionnement indiquant une position et/ou une orientation de l'unité de stimulation (12) par rapport à la partie du corps du patient et/ou par rapport à l'au moins une information d'image concernant la partie du corps du patient afin d'aider ainsi l'utilisateur à positionner l'unité de stimulation (12).

6. Dispositif selon la revendication 5, dans lequel l'au moins une information de positionnement comprend des informations concernant des annotations dans l'espace et/ou des informations concernant des points de cheminement prédéterminés, qui sont fournis de préférence pour le positionnement de l'unité de stimulation (12), et/ou des informations textuelles liées à la position et/ou des informations concernant un nuage d'activation des neurones et/ou des circuits neuronaux à stimuler lié à la position, et/ou des informations liées à la position concernant des neurones et/ou des circuits neuronaux déjà stimulés.

7. Dispositif selon les revendications 3 et 6, dans lequel l'au moins une information de positionnement comprend des informations liées à la position concernant un lieu d'activation et/ou un volume d'activation, le dispositif étant configuré pour déterminer les informations liées à la position sur la base d'une distribution de champ électrique de la bobine magnétique induite spatialement dans le cerveau ; et/ou dans lequel l'au moins une information de positionnement comprend des informations de simulation concernant un lieu d'activation et/ou un volume d'activation, le dispositif étant configuré pour déterminer les informations de simulation sur la base d'un modèle de simulation destiné à simuler la distribution de champ électrique induite.

8. Dispositif selon l'une quelconque des revendications précédentes, dans lequel l'au moins une information d'image comprend des informations concernant une structure anatomique et/ou des informations concernant une gyrification d'une substance blanche du cerveau et/ou des informations d'images cérébrales fonctionnelles.

9. Dispositif selon l'une quelconque des revendications précédentes, dans lequel le dispositif d'affichage (16) est aligné de manière concentrique à un vecteur normal passant par un point focal (20) de l'unité de stimulation (12) par rapport à l'unité de stimulation (12).

10. Dispositif selon la revendication 3 ou 4, dans lequel au moins un repère de position (34) est disposé sur une face supérieure (32) du dispositif d'affichage (16).

11. Dispositif selon l'une quelconque des revendications précédentes, dans lequel le dispositif d'affichage (16) comprend au moins un guide de lumière (36) configuré pour bloquer de la lumière émise obliquement par le dispositif d'affichage.

12. Dispositif selon l'une quelconque des revendications précédentes, dans lequel le dispositif d'affichage (16) comprend un compensateur de parallaxe et/ou un blindage contre les champs magnétiques (38).

13. Dispositif selon l'une quelconque des revendications précédentes, dans lequel le dispositif d'affichage (16) comprend un élément de découplage (40) configuré pour réduire au moins partiellement un couplage capacitif entre l'unité de stimulation (12) et le dispositif d'affichage (16).

14. Dispositif selon les revendications 12 et 13, dans lequel l'élément de découplage (40) est disposé entre le blindage contre les champs magnétiques (38) et un écran du dispositif d'affichage (16).

15. Dispositif selon la revendication 11, dans lequel le dispositif d'affichage (16) comprend au moins un premier et un deuxième guide de lumière (36) configurés pour bloquer de la lumière émise obliquement par le dispositif d'affichage et le premier guide de lumière (36) est disposé au-dessus du deuxième guide de lumière (36) et est tourné par rapport au deuxième guide de lumière de 90° ± 20° relativement à la normale à la surface du deuxième guide de lumière (36).

16. Système de neurostimulation non invasive, le système comprenant au moins une source d'alimentation (102), une unité d'évaluation et de calcul (104) et un dispositif (10) selon l'une quelconque des revendications précédentes.
